# EUROPÄISCHE PATENTSCHRIFT

(11) **EP 3 038 590 B1**
(45) Veröffentlichungstag und Bekanntmachung des Hinweises auf die Patenterteilung: **11.10.2017**
(21) Anmeldenummer: 14752632.1
(22) Anmeldetag: 19.08.2014
(51) Int. Cl.: A61K 8/31, A61K 8/37, A61K 8/39, A61Q 5/02, A61Q 19/10, A61K 8/891, A61K 8/92, A61K 8/81, A61K 8/86, A61K 8/97, A61K 8/33, A61K 8/36

(54) **KOSMETISCHE MITTEL**
COSMETIC COMPOSITION
PRODUIT COSMÉTIQUE

(30) Priorität: 29.08.2013 EP 13182190
(43) Veröffentlichungstag der Anmeldung: 06.07.2016
(73) Patentinhaber: BASF SE, 67056 Ludwigshafen am Rhein (DE)
(72) Erfinder: HLOUCHA, Matthias, 50677 Köln (DE); KÜSTERS, Esther, 40699 Erkrath (DE); SCHORB, Jasmin, 40789 Monheim (DE); NIEENDICK, Claus, 47807 Krefeld (DE); WINZEK, Mirella, 52445 Titz (DE); SEIDLER, Stefanie, 40597 Düsseldorf (DE)
(74) Vertreter: BASF IP Association
(86) Internationale Anmeldenummer: PCT/EP2014/067610
(87) Internationale Veröffentlichungsnummer: WO 2015/028344

(56) Entgegenhaltungen:
- EP-A1- 0 691 127
- EP-A2- 1 955 690
- WO-A1-2006/111256
- DE-A1- 4 428 823
- DE-A1- 19 856 555
- DE-A1-102007 063 134

## Beschreibung

### Gebiet der Erfindung

Die Erfindung liegt auf dem Gebiet der kosmetischen ölhaltigen Mittel, und betrifft Ölkonzentrate, die zur Herstellung derartiger kosmetischer Mittel geeignet sind sowie die kosmetischen Mittel und deren Herstellung.

### Stand der Technik

Kosmetische Mittel werden zur Reinigung und/oder Pflege von Haar und Haut verwendet. Idealerweise werden auch bei der Reinigung von Haut bzw. Haar die durch Waschen verursachten Fett- und Wasserverluste so niedrig wie möglich gehalten, damit Weichheit und Glätte der Haare und/oder Haut möglichst erhalten bleiben. Daher sollen kosmetische Mittel wie Duschgele, Schaumbäder oder Haarshampoos möglichst auch pflegende Anforderungen erfüllen.

In der WO 2008/155075 werden kosmetische Zubereitungen beschrieben, die neben nichtalkoxylierten Tensiden eine Mikroemulsion enthalten aus Alkylpolyglykosid, Glycerinmonoester, einem Ölkörper sowie Wasser. Diese kosmetischen Zubereitungen sind als Haarshampoos mit konditionierender Wirkung geeignet, da sie die Abscheidung von Silikonölen verbessern und verhältnismäßig hohe Mengen an Weichheit und Feuchtigkeit aufweisenden Ölen in Haarshampoos einbringen.

Aus der WO 2011/116881 wiederum sind kosmetische Reinigungsmittel bekannt, die Mikroemulsionen aus Alkyl(oligo)glykosid, Co-tensid, einer nicht-wasserlöslichen organischen Ölkomponente sowie ein Wachs und Wasser enthalten. Diese Mikroemulsionen können ausgenommen alkoxylierter Verbindungen eine Vielzahl von Ölkörpern wie C₆-C₁₀-Fettsäuretriglyceride, pflanzliche Öle oder Kohlenwasserstoffe neben dem Wachs enthalten. Mit Hilfe dieser wachshaltigen Mikroemulsionen kann eine deutlich höhere Deposition von Lipidkomponenten auf der Haut erreicht werden, so dass die negativen Wirkungen von waschaktiven Substanzen wie Hautaustrocknung aufgehoben wird, ohne die positive reinigende Wirkung der Tenside zu verringern.

Aus der Deutschen Offenlegungsschrift DE 10 2007 063 134 ist ein Verfahren zur Herstellung von Öl-in-Wasser Emulsionen bekannt, wonach ein Emulgatorkonzentrat in Kontakt mit einer Ölphase in einem laminaren Strömungsfeld gebracht wird. Dieses Verfahren ermöglicht eine zwar gute Flexibilität hinsichtlich der Art an Emulgatoren sowie der Ölphase, ist aber auf das laminare Strömungsfeld angewiesen.
Aus der Europäischen Patentanmeldung sind flüssige Duschöle bekannt, die im wesentlichen Öl sowie öllösliche flüssige Tenside, vorzugsweise ausgewählt aus der Gruppe der Fettalkoholethersulfate und/oder Fettalkoholetherphospahte enthalten. Derartige Duschöle zeigen keinen voluminösen Schaum.

Verbraucher wünschen sich jedoch reinigende kosmetische Mittel wie Duschgele und Haarshampoos, die einen schönen stabilen, feinteiligen Schaum aufweisen, obgleich für die Haut schützende Ölkomponenten und Wachse enthalten sind, die schaumdämpfend wirken. So sind für viele Verbraucher hohe Mengen an Schaum gleichbedeutend mit einer guten Reinigungsleistung und wenig Schaum verleitet den Verbraucher, unnötig hohe Mengen an Duschgelen bzw. Haarshampoos zu benutzen.

Aufgabe der vorliegenden Erfindung war es, kosmetische ölhaltige Mittel zur Verfügung zu stellen, die neben der reinigenden Wirkung sehr gute Pflegeleistungen für Haut und Haar ermöglichen und trotz schaumdämpfender Ölkomponenten einen voluminösen Schaum aufweisen. Zudem sollen diese ölhaltigen kosmetischen Mittel auf einfache Art und Weise herstellbar sein, möglichst in einem Kaltprozeß.

Überraschenderweise wurde nun gefunden, dass man derartige ölhaltige kosmetische Mittel erhält, wenn man Ölkonzentrate einsetzt, die Alk(en)ylpolyglykolethercitrate als flüssige anionische Tenside, nichtionische Tenside und eine ausgewählte Ölkomponente mit bestimmten Polaritäten enthalten. Diese Ölkonzentrate zeigen überraschenderweise eine nahezu spontane Selbstemulgierung mit Wasser und führen zu feinteiligen O/W-Emulsionen. Der Einsatz eines solchen Ölkonzentrats ermöglicht es, kosmetische Mittel mit hohen Mengen an Öl einfach und stabil herzustellen.

Die erhaltenen erfindungsgemäßen kosmetischen Mittel sind transparent, zeigen eine gute Reinigungsleistung, eine gute Lipid-Deposition, einen guten sensorischen Griff und ein ausgezeichnetes Schaumverhalten, d.h. einen guten und langanhaltenden Schaum. Das Herstellverfahren der kosmetischen ölhaltigen Mittel ist sehr einfach und unproblematisch und kann im Kaltprozeß erfolgen, wobei die kosmetischen Mittel in Form von feinteiligen und stabilen Emulsionen erhalten werden.

### Beschreibung der Erfindung

Ein Gegenstand der vorliegenden Erfindung sind Ölkonzentrate für kosmetische Mittel enthaltend
(a) mindestens ein bei Raumtemperatur flüssiges anionisches Tensid ausgewählt aus der Gruppe der Alk(en)ylpolyglykolethercitrate und
(b) mindestens ein nichtionisches Tensid und
(c) eine Ölkomponente mit
(c1) einem polaren Öl mit einer Polarität zwischen 5 und 30 mN/m und ggf.
(c2) einem unpolaren Öl mit einer Polarität über 35 mN/m,
   wobei das Mischungsverhältnis (c1) : (c1+c2) im Bereich von 0,1 bis 1 liegt,
(d) sowie ggf. Verdickern
Im Sinne der Erfindung sind Ölkonzentrate Mischungen, die in hohen Mengen Öl enthalten, vorzugsweise in Mengen von 30 bis 60 Gew.-% - bezogen auf Ölkonzentrat.

Im Sinne der Erfindung enthalten die Ölkonzentrate bei Raumtemperatur flüssige anionische Alk(en)ylpolyglykolethercitrate als Tenside (a), d.h. sie liegen im Bereich von etwa 10 bis 30 °C als Flüssigkeit vor. Im Sinne der Erfindung sind keine Verdünnungen der anionische Tenside wie wässrige Lösungen, wässrige Gele oder ölige Suspensionen gemeint, sondern die anionischen Verbindungen (a) haben möglichst einen Aktivsubstanzgehalt von praktisch 100 Gew.-%, vorzugsweise mit Aktivsubstanzgehalten zwischen 90 bis 100, bevorzugt 95 bis 100 Gew.-%.

Im Sinne der Erfindung werden als anionische Tenside (a) sowohl Tenside verstanden, die bereits in anionischer Form als Salz vorliegen als auch erst bei Einsatz in wässriger Lösung in die anionische Form überführt werden (dissoziieren). Mit anderen Worten enthalten die anionischen Tenside mindestens eine dissoziierte Gruppe oder werden in wässriger Lösung in diese überführt, ggf. unter Einsatz von pH-Stellmitteln bzw. pH-Regulatien. Daher werden im Sinne der vorliegenden Erfindung unter dem Begriff der anionischen Tenside stets auch die Salze der Alk(en)ylpolyglykolethercitrate verstanden.

Bei den Alk(en)ylpolyglykolethercitraten bzw. deren Salzen kann es sich um Mono-, Di- und/oder Triester der Zitronensäure und alkoxylierten Alkoholen handeln, die der Formel (I) entsprechen: 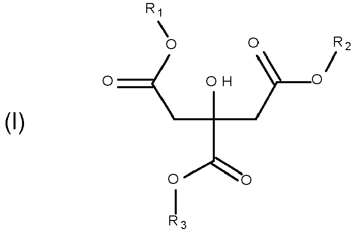 worin
R₁, R₂ und R₃ unabhängig voneinander für Wasserstoff oder den Rest der Formel

   (II) R₄(OCH₂CHR₅)ₙ

   bedeuten, worin
   - R₄: für einen linearen oder verzweigten Alkyl- und/oder Alkenylrest mit 6 bis 22 Kohlenstoff-atomen,
   - R₅: für Wasserstoff oder Methylrest und
   - n: für eine Zahl von 1 bis 20 stehen, mit der Bedingung, dass zumindest einer der Reste R₁, R₂, oder R₃ verschieden von Wasserstoff ist.

Als Salze der Alk(en)ylpolyglykolethercitrate liegen die entsprechenden Carboxygruppen dissoziiert vor.

Bevorzugt im Sinne der Erfindung sind Alk(en)ylpolyglykolethercitrate der Formel (I), worin R₁, R₂ oder R₃ für den Rest der Formel (II) steht und R₄ einen linearen oder verzweigten Alkyl- und/oder Alkenylrest mit 6 bis 22 Kohlenstoffatomen, R5 Wasserstoff und n eine Zahl von 1 bis 20, vorzugsweise 5 bis 10 bedeutet.

Typische Beispiele für den alkoxylierten Alkoholteil der Ester entsprechend Formel (II) sind Additionsprodukte von durchschnittlich 1 bis 20 Mol , vorzugsweise 5 bis 10 Mol Ethylenoxid an Capronalkolohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkcohol, Erucylalkohol and Brassidylalkohol und technische Mischungen davon.

Besonders bevorzugt sind Alk(en)ylpolyglykolethercitrate der Formel (I), worin R₁, R₂ oder R₃ für den Rest der Formel (II) steht und R₄ einen linearen Alkylrest mit 12 bis 14 Kohlenstoffatome, R₅ Wasserstoff und n eine Zahl von 5 bis 10 bedeutet.

Insbesondere bevorzugt sind technische Mischungen von Alk(en)ylpolyglykolethercitrate der Formel (I), worin R₁, R₂ oder R₃ für den Rest der Formel (II) stehen mit der Bedingung, dass in Mengen von mindestens 50 Gew.-%, vorzugsweise 70 Gew.-%, insbesondere 75 bis 80 Gew.-% - bezogen auf Alk(en)ylpolyglykolethercitrate - nur einer der Reste R₁, R₂, oder R₃ verschieden von Wasserstoff ist.

Hervorragend geeignet sind Alkylpolyalkylenglykolethercitrate basierend auf Additionsprodukten von 5 bis 10, insbesondere ungefähr 7 Mol Ethylenoxid an technischen C₁₂-C₁₈, insbesondere C₁₂-C₁₄ Fettalkoholfraktionen. Ganz besonders bevorzugt sind die Polyethylenglykolether des Laurylalkohol, Laureth-7 Citrate, die beispielsweise unter dem Namen Plantapon® LC7 (BASF & Personal Care & Nutrition GmbH) erhältlich sind.

Falls gewünscht können in den erfindungsgemäßen Ölkonzentraten neben den Alk(en)ylpolyglykolethercitraten auch weitere anionische Tenside enthalten sein, die entweder selber bei Raumtemperatur flüssig sind oder dann vorteilhafterweise im Ölkonzentrat gelöst vorliegen. Beispiele für weitere anionische Tenside sind, jeweils in Form ihrer Salze, Ethercarbonsäuren, Acylsarcoside mit 8 bis 24 C-Atomen in der Acylgruppe, Acyltauride mit 8 bis 24 C-Atomen in der Acylgruppe, Acylisethionate mit 8 bis 24 C-Atomen in der Acylgruppe, Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, lineare Alkansulfonate mit 8 bis 24 C-Atomen, lineare Alpha-Olefinsulfonate mit 8 bis 24 C-Atomen, Alpha-Sulfofettsäuremethylester von Fettsäuren mit 8 bis 30 C-Atomen, Alkylsulfate, Alkylpolyglycolethersulfate, Ester der Weinsäure und Zitronensäure, Alkyl- und/oder Alkenyletherphosphate, sulfatierte Fettsäurealkylenglycolester, Monoglyceridsulfate und Monoglyceridethersulfate sowie Kondensationsprodukte aus C₈-C₃₀-Fettalkoholen mit Proteinhydrolysaten und/oder Aminosäuren und deren Derivaten, sogenannte Eiweissfettsäurekondensate, z. B. Lamepon®, Gluadin®, Hostapon® KCG oder Amisoft®.

Die anionischen Tenside (a) können in Mengen von 20 bis 40 Gew.-%, vorzugsweise 25 bis 35 Gew.-% und insbesondere 27 bis 32 Gew.-% - bezogen auf Ölkonzentrat - enthalten sein.

Insbesondere sind in den erfindungsgemäßen Ölkonzentraten ausschließlich Alk(en)ylpolyglykolethercitrate, vorzugsweise der Formel (I), als anionisches Tensid (a) enthalten.

Ein weiterer Bestandteil der erfindungsgemäßen Ölkonzentrate sind (b) nichtionische Tenside enthalten. Beispiele für nichtionische Tenside (b) sind:
Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
Alkyl- und/oder Alkenyloligoglykoside mit 8 bis 22 Kohlenstoffatomen im Alk(en)ylrest und deren ethoxylierte Analoga;
Anlagerungsprodukte von 1 bis 15 Mol Ethylenoxid an Ricinusöl und/oder gehärtetes Ricinusöl; Anlagerungsprodukte von 15 bis 60 Mol Ethylenoxid an Ricinusöl und/oder gehärtetes Ricinusöl;
Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
Partialester von Polyglycerin (durchschnittlicher Eigenkondensationsgrad 2 bis 8), Poly-ethylenglycol (Molekulargewicht 400 bis 5000), Trimethylolpropan, Zuckeralkoholen (z.B. Sorbit), Alkylglycosiden (z.B. Methylglucosid, Butylglucosid, Laurylglucosid) sowie Polyglycosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
Weitere Beispiele für nicht-ionische Tenside sind:
   Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin; Mono-, Di- und Trialkylphosphate sowie Mono-, Di- und/oder Tri-PEGalkylphosphate und deren Salze; Wollwachsalkohole; Polysiloxan-Polyalkyl-Polyether-Copolymere bzw. entsprechende Derivate; Block-Copolymere z.B. Polyethylenglycol-30 Dipolyhydroxystearate; Polymeremulgatoren, Polyalkylenglycole und/oder Glycerincarbonat.

Bevorzugte nichtionische Tenside (b) sind Anlagerungsprodukte von Ethylenoxid und/oder Propylenoxid an Fettalkohole, Fettsäuren, Alkylphenole oder an Ricinusöl, Partialester von Glycerin, Partialester von Sorbitan, Alkyl- und/oder Alkenyloligoglycoside und/oder Ester von Polyglycerinen.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Typische Beispiele für Partialester von Glycerin sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremonoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Partialester von Sorbitan kommen in Frage Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Auch Alkyl- und/oder Alkenyloligoglycoside sind geeignete nichtionische Tenside, die besonders hautfreundlich sind. Die Verbindungen und ihre Herstellung sind aus dem Stand der Technik bekannt und erfolgt insbesondere durch Umsetzung von Glucose oder Oligosacchariden mit primären Alkoholen mit 8 bis 22 C-Atomen, vorzugsweise 12 bis 22, und besonders bevorzugt 12 bis 18 C-Atomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare® zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nichtionische Tenside geeignet.

Ganz besonders bevorzugt sind als nichtionische Tenside in den erfindungsgemäßen Ölkonzentraten (b) Ester von Polyglycerinen enthalten. Die Säurekomponente dieser Polyglycerinester kann sich von geradkettigen, verzweigten, gesättigten und/oder ungesättigten Carbonsäuren ggf. mit funktionellen Gruppen wie Hydroxylgruppen ableiten. Besonders bevorzugt handelt es sich bei der Säurekomponente um Isostearinsäure und/oder Poly-12-hydroxystearinsäure.

Einer Ausführungsform entsprechend erwies sich als nichtionisches Tensid (b) das Polyglyceryl-3-Diisostearat, welches von der BASF Personal Care and Nutrition GmbH unter der Bezeichnung Lameform^{®} TGI vermarktet wird, als gut geeignet Ölkonzentrate herzustellen, die leicht in Wasser selbstemulgierbar sind.

Nach einer besonders bevorzugten weiteren Ausführungsform erwiesen sich Ester von Poly-12-hydroxystearinsäure mit Polyglycerinen und insbesondere von Polyglycerinen mit folgender Homologenverteilung als vorteilhaft (bevorzugte Mengen sind in Klammern angegeben):

| | | |
|---|---|---|
| Glycerine | : 5 bis 35 (15 bis 30) | Gew.-% |
| Diglycerine | : 15 bis 40 (20 bis 32) | Gew.-% |
| Triglycerine | : 10 bis 35 (15 bis 25) | Gew.-% |
| Tetraglycerine | : 5 bis 20 (8 bis 15) | Gew.-% |
| Pentaglycerine | : 2 bis 10 (3 bis 8) | Gew.-% |
| Oligoglycerine | : ad 100 | Gew.-% |

Ganz besonders geeignet ist der Poly(12-hydroxystearinsäure)polyglycerinester der beispielsweise von der BASF Personal Care and Nutrition GmbH unter der Bezeichnung Dehymuls^{®} PGPH vermarktet wird.

In den erfindungsgemäßen Ölkonzentraten sind die nichtionischen Tenside (b), in Mengen von 10 bis 30 Gew.-%, vorzugsweise 15 bis 25 Gew.-% enthalten.

Des Weiteren enthalten die erfindungsgemäßen Ölkonzentrate eine Ölkomponente. Die Ölkomponente enthält zwingend ein polares Öl (c1) entweder als alleinige Ölkomponente oder in Mischung mit einem unpolaren Öl (c2).

Mit anderen Worten: die erfindungsgemäßen Ölkonzentrate enthalten die Ölkomponente (c) mit einem polaren Öl (c1) mit einer Polarität zwischen 5 und 30 mN/m und ggf. einem unpolaren Öl (c2) mit einer Polarität über 35 mN/m ist, wobei das Mischungsverhältnis von (c1) : (c1+c2) im Bereich von 0,1 bis 1 liegt.

Unter Mischungsverhältnis ist das Verhältnis der Masse von (c1) im Verhältnis zur Masse von (c1+c2) zu verstehen. Sofern kein unpolares Öl enthalten ist (c2 = 0), entspricht das Mischungsverhältnis von (c1) : (c1+c2) dem Wert 1, d.h. nur das polare Öl (c1) ist zugegen.

Der Begriff "Öl" steht in der vorliegenden Beschreibung für ein wasserunlösliches, organisches, natürliches oder synthetisches, kosmetisch geeignetes Öl, welches vorzugsweise eine flüssige bzw. viskose Konsistenz bei 23 °C aufweist. "Wasserunlöslich" meint in diesem Zusammenhang eine Wasserlöslichkeit des Öls von höchstens 2 Gew.-% bei 20 °C.

Im Sinne der vorliegenden Erfindung enthält die Ölkomponente (c) mindestens ein polares Öl (c1) mit einer Polarität zwischen 5 und 30 mN/m, vorzugsweise 15 bis 28 mN/m.

Es hat sich als vorteilhaft erwiesen, wenn die Ölkomponente (c) ein weiteres, unpolares Öl (c2) mit einer Polarität über 35 mN/m, vorzugsweise zwischen 35 und 60mN/m, enthält.

In einer bevorzugten Ausführungsform hat die Ölkomponente (c) ein Mischungsverhältnis von (c1) : (c1+c2) von 0,3 bis 0,7 bevorzugt von 0,4 bis 0,6 und insbesondere von etwa 0,45 bis 0, 55.

Die Polarität eines Öls ist definiert als Polaritätsindex (Grenzflächenspannung) des Öls gegen Wasser. Die Polarität wurde bestimmt unter Einsatz eines Ringtensiometers (e.G. Krüss K 10), der die Grenzflächenspannung in mN/m misst in Analogie der ASTM Methode D971-99a (2004).

Im Sinne der vorliegenden Erfindung werden die Öle mit einer Polarität über 35 mN/m als unpolar und mit einer Polarität zwischen 5 und 30 mN/m als polar bezeichnet.

Geeignete polare Öle (c1) sind beispielsweise Guerbetalkohole auf Basis von Fettalkoholen mit 6 bis 18, vorzugsweise 8 bis 10 Kohlenstoffatomen (z. B. Eutanol^{®} G), Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat (Cetiol J600®), Behenylmyristat, Behenylpalmitat, Behenylstearat, Behenylisostearat, Behenyloleat, Behenylbehenat, Behenylerucat, E-rucylmyristat, Erucylpalmitat, Erucylstearat, Erucylisostearat, Erucyloleat, Erucylbehenat und Erucylerucat in Betracht. Daneben eignen sich Ester von linearen C₆-C₂₂-Fettsäuren mit verzweigten Alkoholen, insbesondere 2-Ethylhexanol, Ester von C₃-C₃₈-Alkylhydroxy-carbonsäuren mit linearen oder verzweigten C₆-C₂₂-Fettalkoholen, insbesondere Diethylhexylmalat, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis von C₆-C₁₀-Fettsäuren, flüssige Mono-/Di-Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxyl-gruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C-Atomen, Ester der Benzoesäure mit linearen und/oder verzweigten C₆-C₂₂-Alkoholen (z. B. Finsolv^{®} TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen (Hydagen^{®} HSP, Sovermol® 750, Sovermol^{®} 1102).

Einer Ausführungsform der vorliegenden Erfindung entsprechend werden als polare Öle (c1) Mono-, Di und Trifettsäureester von Glycerin eingesetzt, die vorzugsweise 6 bis 24 und insbesondere 8 bis 18 Kohlenwasserstoffatome aufweisen und gesättigt und/oder ungesättigt sein können und durch chemische Synthese aus einer natürlichen (pflanzlichen oder tierischen) Quelle erhalten werden. Bevorzugt sind hiervon Verbindungen, die sich von pflanzlichen Quellen ableiten und insbesondere Glycerinester von Fettsäuren mit 8 und/oder 10 Kohlenstoffatome, vorzugsweise Di- und/oder Triglyceridester wie sie erhältlich sind unter dem Handelsnamen Myritol® 312 von der BASF Personal Care & Nutrition GmbH (INCI name: Caprylic/Capric triglyceride).

Einer weiteren Ausführungsform der vorliegenden Erfindung entsprechend werden als polare Öle (c1) die in der kosmetischen Industrie weithin bekannten pflanzlichen Öle wie Erdnussöl, Rizinusöl, Kokosnussöl, Maisöl, Olivenöl, Palmkernöl, Sonnenblumenöl, Sojaöl, Rapsöl, Mandelöl, Traubenkernöl, Distelöl, Weizenkeimöl, Nachtkerzenöl, Macadamianussöl, Arganöl, Avocadoöl und dergleichen mehr eingesetzt.

Insbesondere geeignet sind im Sinne der Erfindung Ölkonzentrate mit polaren Ölen (c1) ausgewählt aus der von Olivenöl, Sojaöl, Sonnenblumenöl, Mandelöl, Arganöl und/oder Avocadoöl gebildeten Gruppe.

Als unpolare Öle (c2) können die weithin in der kosmetischen Industrie bekannten Öle eingesetzt werden, vorzugsweise Kohlenwasserstoff basierte Öle wie aliphatische und/oder aromatische Öle, die vorzugsweise 8 bis 32 , insbesondere 15 bis 20 Kohlenstoffatome aufweisen. Beispiele sind Squalan, Squalen, Paraffinöle, Isohexadecan, Isoeicodecan, Polyolefine wie Polydecene, hydrogenierte Polyisobutene, Dialkylcyclohexan und Mineralöl. Bevorzugt sind Paraffinöle wie die dünnflüssigen Paraffine (*Paraffinum perliquidum*)*,* die eine Viskosität von 25 bis 80 mPa·s haben und/oder die dickflüssigen Paraffine (*Paraffinum subliquidum*)*,* die als ölige Flüssigkeit eine Viskosität von 110 bis 230 mPa·s aufweisen. Weiterhin bevorzugt sind die hydrogenierten Polyisobutene, die beispielsweise unter dem Handelsnamen Luvitol® Lite von der BASF SE erhältlich sind. Hervorzuheben im Sinne der Erfindung sind Paraffinöle und/oder hydrogenierte Polyisobutene als unpolare Öle (c2).

Im Sinne der vorliegenden Erfindung wird als Ölkomponente (c) mit sehr gutem Erfolg eine Mischung aus
(c1) pflanzlichen Ölen und
(c2) unpolaren Kohlenwasserstoff basierten Ölen und insbesondere eine Mischung aus
(c1) pflanzlichen Ölen und
(c2) Paraffinölen eingesetzt.

In einer bevorzugten Ausführungsform der vorliegenden Erfindung wird als Ölkomponente (c) eine Mischung aus Mono-, Di und/oder Trifettsäureester von Glycerin mit Fettsäuren mit 8 bis 10 Kohlenstoffatomen als (c1) und Paraffinöl als (c2) eingesetzt.

Nach einer zweiten bevorzugten Ausführungsform wird als Ölkomponente (c) eine Mischung aus Sonnenblumenöl als (c1) und hydriertem Polyisobutene als (c2) eingesetzt.

In der folgenden Tabelle sind die Polaritäten der typischen, meist genutzten Öle aufgeführt.

| **Öl (CTFA Name)** | **Polaritäts Index [mN/m]** |
|---|---|
| Unpolar | |
| Isoparaffin (C₁₂-C₁₄) | 53.0 |
| Squalan® | 46.2 |
| Isohexadekan (ARLAMOL® ND) | 43.8 |
| Mineral Öl (Paraffin oil perliquidum) | 43.7 |
| Mineral Öl (Paraffin oil subliquidum) | 38.3 |

| Polar | |
|---|---|
| Oleyl Erucate (Cetiol J600®) | 27,1 |
| Passiflora Incarnata Oil (Cegesoft® PFO) | 27,2 |
| Rapsöl | 21,9 |
| Myritol® 312 | 25,3 |
| Cetystearyloctanoat | 28.6 |
| Dimethicon (silicon oil 20 ct) | 26.6 |
| Isopropylpalmitat | 25.2 |
| Octyldodecanol | 24.8 |
| Dioctyladipate (ARLAMOL® DOA) | 24.5 |
| Isopropymyristat | 24.2 |
| Octylpalmitat (2-ethylhexylpalmitat) | 23.1 |
| Hexamethyldisiloxan | 22.7 |
| Isopropylstearat | 21.9 |
| Carpyl/Caprin -Triglyceride | 21.3 |
| Isopropylisostearat | 21.2 |
| Jojoba Öl | 20.8 |
| Cyclomethicone (ARLAMOL® D4) | 20.6 |
| Erdnussöl | 20.5 |
| Mandelöl | 20.3 |
| Sonnenblumenöl | 19.3 |
| Decyloleat | 18.7 |
| Avocadoöl | 18.3 |
| Olivenöl | 16.9 |
| Rizinusöl | 13.7 |
| Calendula Öl | 11.1 |
| Weizenkeimöl | 8.3 |

Weitere geeignete Öle sind in der DE 102004003436 A1 beschrieben, die hier miteinbezogen werden:

| **Öl (CTFA Name)** | **Polaritäts Index [mN/m]** |
|---|---|
| unpolar | |
| Hydrogenated Polylsobutene (Luvitol Lite®) | 44.7 |
| Isohexadecane | 43.8 |
| Mineral Oil | 43.7 |
| Isoeikosan | 41.9 |
| Dioctylcyclohexane | 39.0 |
| | |

| polar | |
|---|---|
| Dicaprylyl Carbonate | 31.7 |
| Dicaprylyl Ether | 30.9 |
| Dihexyl Carbonate | 30.9 |
| Jojobaöl Gold | 26.2 |
| Octyl Palmitate | 23.1 |
| Macadamia Nut Oil | 22.1 |
| Isopropyl Stearate | 21.9 |
| Dibutyl Adipate | 14.3 |
| Propylene Glycol Monoisostearate | 6.2 |
| Cocoglycerides | 5.1 |

Besonders bevorzugt im Sinne der Erfindung ist auch Sojabohnenöl, das mit einem Polaritätsindex 13,5 mN/m als polar zu bezeichnen ist.

Im Sinne der Erfindung ist die Ölkomponente (c) vorzugsweise in Mengen von 30 bis 60 Gew.-%, bevorzugt von 40 bis 55 Gew.-% - bezogen auf Ölkonzentrat - enthalten.

Im Sinne der Erfindung ist es möglich, dass in der Ölkomponente (c) auch Wachse enthalten sind, wobei deren Anteil vorzugsweise unter 20, insbesondere unter 15 Gew.-% - bezogen auf Ölkomponente - beträgt.

Wachse im Sinne der Erfindung sind natürliche Substanzen tierischer oder pflanzlicher Herkunft dar, welche bei Raumtemperatur (21 °C) fest sind jedoch im Allgemeinen eine gewisse Verformbarkeit besitzen. Wachse sind in Wasser unlöslich, jedoch in Ölen löslich und zur Bildung von wasserabweisenden Filmen befähigt.

Typische Beispiele für Wachse im Sinne der vorliegenden Lehre sind natürliche Wachse, wie z.B. Shorea Stenoptera Butter (cegesoft SH), Sheabutter, Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse. Bevorzugt sind die natürlichen Wachse, insbesondere bevorzugt Shea-butter (auch Sheafett, Karitefett oder Caritefett, Galambutter) , welches ein natürlicher fester Fettstoff ist, der aus der Pflanze Butyrospermum parkii, dem afrikanischen Sheabutterbaum, gewonnen wird und in kommerziellen Mengen verfügbar ist. Ihr Schmelzbereich liegt bei 35 bis 42 °C. Üblicherweise enthält Sheabutter 89 bis 98 Gew.-% Triglyceride, Glycerinpartialester und freie Fettsäuren sowie einen Gehalt von 2 bis 11 Gew.-% unverseifbarer Anteile, von denen Kohlenwasserstoffe (Karitene), Triterpenalkohole und Sterole die wichtigsten sind.

Des Weiteren kann in den erfindungsgemäßen Ölkonzentraten ein Verdicker als weitere optionale Komponente d) enthalten sein , was sich vorteilhaft auf die Viskositäten der kosmetischen Mittel, in die sie eingebracht werden, auswirkt.

Geeignete Verdicker d) sind Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate und hydrophob modifizierte Polyacrylate, Polyacrylamide, Polymere, Polyvinylalkohol und Polyvinylpyrrolidon. Als besonders wirkungsvoll haben sich auch Bentonite erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, ethoxylierte und/oder propoxylierte Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Bevorzugt enthalten die Ölkonzentrate als d) Mischungen aus ethoxylierten und/oder propoxylierten Polyolestern des Trimethylolpropans und ethoxylierten Fettalkoholen und insbesondere eine Mischung aus einem ethoxylierten und propoxylierten Trimethyloleat und einem ethoxyliertem Laurylalkohol, welches unter dem Handelsnamen Arlypon® TT von der BASF Personal Care & Nutrition GmbH erhältlich ist.

Insbesonders geeignete Ölkonzentrate enthalten
(a) Alk(en)ylpolyglykolethercitrate in Mengen von 20 bis 40 Gew.-%, vorzugsweise 25 bis 35 Gew.-% und
(b) mindestens ein nichtionisches Tensid in Mengen von 10 bis 30 Gew.-%, vorzugsweise 15 bis 25 Gew.-% sowie die
(c) Ölkomponente in Mengen von 30 bis 60 Gew.-%, vorzugsweise 40 bis 55 Gew-% sowie ggf.
(d) Verdicker in Mengen von 0 bis 15 Gew.-%.

Ein ganz besonders geeignetes Ölkonzentrat nach der vorliegenden Erfindung besteht aus 25 bis 35 Gew.- % Alkylpolyglykolethercitrat, vorzugsweise Laureth-7 Citrat 15 bis 25 Gew.-% Ester von Polyglycerinen, insbesondere Poly-12-hydroxystearinsäure 40 bis 55 Gew.-% Ölkomponente (c) mit einer Mischung aus Mono-, Di und/oder Trifettsäureester von Glycerin mit Fettsäuren mit 8 bis 10 Kohlenstoffatomen als (c1) und Paraffinöl als (c2) im Mischungsverhältnis(c1) : (c1+c2) von 0, 45 bis 0, 55.

Ein weiteres ganz besonders geeignetes Ölkonzentrat der vorliegenden Erfindung besteht aus 25 bis 35Gew.-% Alkylpolyglykolethercitrat, vorzugsweise Laureth-7 Citrat 15 bis 25Gew.-% Ester von Polyglycerinen, insbesondere Poly-12-hydroxystearinsäure 35 bis 50Gew.-% Ölkomponente (c) mit einer Mischung aus Sonnenblumenöl als (c1) und hydriertem Polyisobutene als (c2) im Mischungsverhältnis (c1) : (c1+c2) von 0, 45 bis 0, 55 sowie
5 bis 15 Gew.-% Verdicker, vorzugsweise einer Mischung aus einem ethoxylierten und propoxylierten Trimethyloleat und einem ethoxyliertem Laurylalkohol.

Die erfindungsgemäßen Ölkonzentrate können im Labormaßstab durch einfaches, manuelles Rühren bei Raumtemperatur oder, bei leicht erhöhten Temperaturen, vorzugsweise unter 60 °C, hergestellt werden. Vorzugsweise wird die Ölkomponente (c) vorgelegt und das flüssige anionische Tensid Alk(en)ylpolyglykolethercitrate (a) sowie das nichtionische Tensid (b) und ggf. der Verdicker (d) eingerührt. Zusätzliches Wasser, welches nicht in Form von wässrigen Formulierungen der Tenside und/oder Verdicker eingebracht werden, kann im letzten Verfahrensschritt zuzugeben werden, ist aber im Sinne der Erfindung nicht erwünscht. Vielmehr werden Ölkonzentrate gewünscht, die praktisch kein Wasser enthalten, vorzugsweise 0 bis 5 Gew.-%, vorzugsweise unter 3 Gew.-% und insbesondere zwischen 0 Gew.-% und 2 Gew.-%.

Die erfindungsgemäßen Ölkonzentrate sind klar und weisen niedrige Viskositäten auf. Die erfindungsgemäßen Ölkonzentrate zeigen eine sehr hohe Lagerstabilität von mehreren Wochen.

Die erfindungsgemäßen Ölkonzentrate können problemlos mit Wasser vermischt werden, vorzugsweise gibt man die Konzentrate zu vorgelegtem Wasser bei Raumtemperatur, wobei sich spontan ohne zusätzlichen mechanischen Energieeintrag (wie Rühren) sehr feinteilige O/W-Emulsionen ausbilden.

Somit lassen sich die erfindungsgemäßen Ölkonzentrate problemlos in wasserhaltige kosmetische Mittel einbringen, wodurch kosmetische Mittel mit einem hohen Ölanteil entstehen. Über den Weg der erfindungsgemäßen Ölkonzentrate können Öle in die kosmetische Mittel ohne aufwendiges, scherintensives Rühren im Rahmen eines Kaltprozesses, also ohne hohe Temperaturen, eingebracht werden. Darüber hinaus sind die so hergestellten ölhaltigen kosmetischen Mittel transparent, sehr feinteilig mit Teilchengrößen unter 1 µm und lagerstabil. Als zusätzlicher großer Vorteil erweist sich, dass die ölhaltigen kosmetischen Mittelweiterhin gute Schaumeigenschaften zeigen.

Im Sinne der vorliegenden Erfindung werden die erfindungsgemäßen Ölkonzentrate vorzugsweise in kosmetischen Reinigungsmittel eingesetzt werden, insbesondere in Haarshampoos, Haarlotionen, Schaumbäder, Duschbäder, Duschgele, Duschcremes und kombinierten Haar- und Duschshampoos.

Ein weiterer Gegenstand der vorliegenden Erfindung ist ein Verfahren zur Herstellung von ölhaltigen kosmetischen Mittel in einem Kaltprozess, also bei Raumtemperatur, vorzugsweise im Bereich von 20 bis 25 °C, wobei die erfindungsgemäßen Ölkonzentrate mit B) anionischen Tensiden und C) nichtionischen Tensiden und D) kosmetischen Zusatzstoffen und E) Wasser verrührt werden.

Dabei können die erfindungsgemäßen Ölkonzentrate in die vorgelegten Komponenten B), C), D) und/oder E) eingebracht werden. Die Komponenten können entweder alle oder nur teilweise sowie insgesamt oder nur anteilsweise vorgelegt werden. Einer Möglichkeit entsprechend kann das erfindungsgemäße Ölkonzentrat in vorgelegtes Wasser E) eingebracht werden und zu dem so erhaltenen Premix werden die weiteren Komponenten der kosmetischen Mittel B) und C) und D) gegeben. Es hat sich als vorteilhaft erwiesen, wenn nur Anteile der gesamten Wassermenge E) vorgelegt, und die Restmenge an Wasser erst nach dem Einbringen der anderen Komponenten zugegeben wird.

Die Einbringung der erfindungsgemäßen Ölkonzentrate in die kosmetischen Mittel wird vorzugsweise durch kurzes Umrühren bzw. durchrühren unterstützt.

Die kosmetischen Mittel enthalten neben
A) den erfindungsgemäßen Ölkonzentraten als weitere Komponenten
B) anionische Tenside
C) weitere von B) verschiedene Co-Tenside
D) kosmetische Zusatzstoffe und
E) Wasser.

Überraschenderweise zeigen kosmetische Mittel, die die Ölkonzentrate vorformuliert enthalten, verbesserte Eigenschaften, insbesondere im Hinblick auf Aussehen und Lagerstabilität. So ist auch nach längerer Lagerung der erfindungsgemäßen kosmetischen Mittel keine Trennung des Öls zu beobachten wie es beobachtet wird, wenn Öl direkt, d.h. ohne Vorformulierung als Konzentrat, in die kosmetischen Mittel eingebracht wird.

Vorzugsweise enthalten die kosmetischen Mittel
A) 0,5 bis 20 Gew.-% erfindungsgemäße Ölkonzentrate
B) 5,0 bis 20 Gew.-% anionische Tenside
C) 1 bis 15 Gew.-% weitere von B) verschiedene Co-Tenside
D) 0,1 bis 10 Gew.-% kosmetische Zusatzstoffe und
E) ad 100 Gew.-% Wasser.

Als anionische Tenside B) können solche in den kosmetischen Mitteln enthalten sein, die ausgewählt sind aus von Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid-(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate gebildeten Gruppe. Besonders bevorzugt sind dabei die Fettalkoholethersulfate wie beispielsweise Sodiumlaurylethersulfat oder andere Verbindungen mit vergleichbarem Schaumverhalten. Alkylethersulfate ("Fettalkoholethersulfate") stellen bekannte anionische Tenside dar, die großtechnisch durch SO₃- oder Chlorsulfonsäure (CSA)-Sulfatierung von Fettalkohol- oder Oxoalkoholpolyglycolethern und nachfolgende Neutralisation hergestellt werden. Im Sinne der Erfindung kommen Ethersulfate in Betracht, die der Formel (II) folgen,R²O-(CH₂CH₂O)ₘSO₃X (II) in der R₂ für einen linearen oder verzweigten Alkyl- und/oder Alkenylrest mit 6 bis 22 Kohlenstoffatomen, n für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen.

Besonders bevorzugt ist der Einsatz von Ethersulfaten auf Basis von Addukten von durchschnittlich 2 bis 3 Mol Ethylenoxid an technische C₁₂/₁₄- bzw. C₁₂/₁₈-Kokosfettalkoholfraktionen in Form ihrer Natrium- und/oder Magnesiumsalze. Bevorzugte anionische Tenside B) sind aus der von Fettalkoholethersulfat, Monoglycerid(ether)sulfat, Mono- und Dialkylsulfosuccinat, Ethercarbonsäuren bzw. deren Salze, Acylglutamate und Alkyloligoglucosidsulfate gebildeten Gruppe.

Besonders bevorzugt enthalten die kosmetischen Mittel
A) 5,0 bis 15 Gew.-% erfindungsgemäße Ölkonzentrate
B) 5,0 bis 20 Gew.-% anionische Tenside aus der von Fettalkoholethersulfat, Monoglycerid(ether)sulfat, Mono- und Dialkylsulfosuccinat, Ethercarbonsäuren bzw. deren Salze, Acylglutamate und Alkyloligoglucosidsulfate gebildeten Gruppe
C) 1 bis 10 Gew.-% weitere von B) verschiedene Co-Tenside
D) 0,5 bis 5 Gew.-% kosmetische Zusatzstoffe und
E) ad 100 Gew.-% Wasser.

Als Co-tenside C), die von B) verschieden sind, eigenen sich prinzipiell nichtionische, amphotere und/oder kationische Tenside.

Typische Beispiele für nichtionische Tenside sind bereits bei den Ölkonzentraten als Komponente b) beschrieben worden, auf die hiermit ausdrücklich Bezug genommen wird im Hinblick auf die Offenbarung. Geeignete nichtionische Tenside sind Fettalkoholpolyglycolether, AIkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate, Alkyl- und/oder Alkenyloligoglycoside und Aminoxide. Sofern die nichtionischen Tenside Polyglycoletherketten enthalten, können diese eine konventionelle, vorzugsweise jedoch eine eingeengte Homologenverteilung aufweisen.

Im Sinne der Erfindung bevorzugt sind als weitere Co-Tenside C) amphotere bzw. zwitterionische Tenside.

Daher sind kosmetische Mittel bevorzugt, die
A) 5,0 bis 15 Gew.-% erfindungsgemäße Ölkonzentrate
B) 5,0 bis 20 Gew.-% anionische Tenside ausgewählt aus der von Fettalkoholethersulfat, Monoglycerid(ether)sulfat, Mono- und Dialkylsulfosuccinat, Ethercarbonsäuren bzw. deren Salze, Acylglutamate und Alkyloligoglucosidsulfate gebildeten Gruppe
C) 1 bis 10 Gew.-% amphotere und/oder kationische Tenside
D) 0,5 bis 5,0 Gew.-% kosmetische Zusatzstoffe sowie
E) ad 100 Gew.-% Wasser enthalten.

Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze.

Typische Beispiele für amphotere bzw. zwitterionische Tenside sind Alkylbetaine, Alkylamidobetaine, Aminopropionate, Aminoglycinate, Imidazoliniumbetaine und Sulfobetaine, besonders bevorzugt ist Cocamidopropylbetain. Bei den genannten Tensiden handelt es sich ausschließlich um bekannte Verbindungen. Typische Beispiele für besonders geeignete milde, d.h. besonders hautverträgliche Tenside sind Fettsäureglucamide, Alkylamidobetaine wie Coco-amidopropylbetain, Amphoacetate wie Sodiumcocoamphoacetat und/oder Proteinfettsäurekondensate, letztere vorzugsweise auf Basis von Weizenproteinen. Besonders bevorzugt ist Cocoamidopropylbetain als C) enthalten.

Die kosmetischen Zusatzstoffe D) können ausgewählt sein aus der Gruppe, die gebildet wird von Hydrotope wie Glycerin, Konservierungsmittel, Zitronensäure, Phenoxyethanol, UV-Lichtschutzfilter, Antioxidantien, biogene Wirkstoffe, Parfum, Farbstoffe, Biozide, Entschäumer, und pH-Regulantien.

Bevorzugt bei Einsatz von Ölkonzentraten auf Basis von Alkylpolyalkylenglykolethercitrate als flüssiges Tensid a) zur Herstellung von kosmetischen Mitteln sind pH-Regulantien. Für kosmetische Mittel wird dabei vorzugsweise ein pH-Bereich 4 - 8 eingestellt.

Weitere übliche kosmetische Zusatzstoffe D) sind beispielsweise Emulgatoren, Perlglanzwachse, Stabilisatoren, Salz, Verdickungsmittel, Konsistenzgeber, Selbstbräuner, Pigmente, Antioxidationsmittel, Antischuppenmittel, Filmbildner, Quellmittel, Insektenrepellentien, Deodorant- und Antitranspirantwirkstoffe, biogene Wirkstoffe. Als biogene Wirkstoffe sind dabei insbesondere Tocopherol, Tocopherolacetat, Tocopherolpalmitat, Desoxyribonucleinsäure, Coenzym Q10, Ascorbinsäure, Retinol- und Retinylderivate, Bisabolol, Allantoin, Phytantriol, Panthenol, AHA-Säuren, Aminosäuren, Ceramide, essentielle Öle, Hyaluronsäure, Creatin, Proteinhydrolysate, Pflanzenextrakte, Peptide und Vitaminkomplexe bevorzugt.

Für den Einsatz als reinigendes Gel oder als Paste und Salbe sind Konsistenzgeber und Verdickungsmittel als kosmetische Zusatzstoffe D) bevorzugt enthalten. Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Poly-ethylenglycolmono- und -diester von Fettsäuren, Polyacrylate und hydrophob modifizierte Polyacrylate, Polyacrylamide, Polymere, Polyvinylalkohol und Polyvinylpyrrolidon. Als besonders wirkungsvoll haben sich auch Bentonite erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Falls Konservierungsmittel erfindungsgemäß als Zusatzstoff D) eingesetzt werden, sind diese bevorzugt ausgewählt aus der Gruppe, die gebildet wird von Benzoesäure und deren Salze, Zitronensäure und deren Salze, Phenoxyethanol, Benzylalkohol, Alkylparabenen, bevorzugt Ethyl-, Methyl- und Propylparaben. Als Konservierungsmittel eignen sich weiterhin beispielsweise Formaldehydlösung, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als kosmetische Zusatzstoffe D) sind auch kationische Verbindungen vorteilhaft, die der Haut ein angenehmes Glättegefühl und Weichheit geben. Insbesondere geeignet sind quaternäre Ammoniumverbindungen oder ein kationische Polymere enthalten. Unter quaternären Ammoniumverbindungen werden hierbei insbesondere quaternierte Fettsäuretriethanolaminestersalze verstanden. Geeignet sind aber ebenso Alkylammoniumhalogenide. Geeignete kationische Polymere sind beispielsweise kationische Cellulosederivate, wie z.B. eine quaternierte Hydroxyethylcellulose, kationische Stärke, Copolymere von Diallylammoniumsalzen und Acrylamiden, quaternierte Vinylpyrrolidon/Vinyl-imidazol-Polymere, wie z.B. Luviquat (BASF), Kondensationsprodukte von Polyglycolen und Aminen, quaternierte Kollagenpolypeptide, wie beispielsweise Lauryldimonium hydroxypropyl hydrolyzed collagen (Lamequat), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine), Copolymere der Acrylsäure mit Dimethyldiallyl-ammoniumchlorid (Merquat 550), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, quaternierte Ammoniumsalz-Polymere, wie z.B. Mirapol A-15, Mirapol AD-1, Mirapol AZ-1 der Firma Miranol.

Weitere bevorzugte kationische Polymere sind gewählt aus der Gruppe der Homo- oder Copolymere von Ester- oder Amidderivaten der Acryl- oder Methacrylsäure (z.B. INCI: Polyquaternium-7, oder PQ-7), Homopolymeren aus Methacryloylethyltrimethylammonium Chlorid (INCI: Polyquaternium-37, oder PQ-37), quaternären Copolymeren aus Hydroxyethylcellulose und Diallyl-dimethyl-ammoniumchlorid (INCI: Polyquaternium-4, oder PQ-4), polymeren quaternisierten Ammoniumsalzen von Hydroxyethylcellulose, welche mit einem trimethylammonium-substituierten Epoxid modifiziert sind (INCI: Polyquaternium-10, oder PQ-10), depolymerisierten Guar Gum Derivaten, welche quaternisiert sind (INCI: Guar Hydroxypropyl Trimonium Chlorid) oder quaternisierte Guarderivate und quaternären Copolymeren aus Hydroxyethylcellulose und Diallyl-demethylammoniumchlorid. In einer bevorzugten Ausführungsform wird das kationische Polymer ausgewählt aus der Gruppe, die gebildet wird von Polyquaternium-7, Polyquaternium-10 und kationischen Guar-Derivaten. Weiterhin können mit Vorteil kationische Polymere gemäß der Lehre der EP 1 767 554 A1 Verwendung finden, die seitens der Anmelderin unter der Marke Polyquart Pro vertrieben werden. Die erfindungsgemäßen kosmetischen Mittel enthalten vorzugsweise 0,05 bis 2 Gew.-% dieser kationischen Polymere.

Einer insbesondere geeigneten Ausführungsform der vorliegenden Erfindung entsprechend enthalten die kosmetischen Mittel
A) 5,0 bis 15 Gew.- % erfindungsgemäße Ölkonzentrate
B) 5,0 bis 15 Gew.-% Fettalkoholethersulfat
C) 2 bis 10 Gew.-% Cocoamidopropylbetain
D) 0,5 bis 5 Gew.-% kosmetische Zusatzstoffe ausgewählt aus der Gruppe der Konservierungsmittel und Verdicker, vorzugsweise Mischungen aus ethoxylierten und / oder propoxylierten Polyolestern des Trimethylolpropans und ethoxylierten Fettalkoholen als Verdicker sowie
E) ad 100 Gew.-% Wasser.

Durch Einarbeitung des Ölkonzentrats als Komponente A) in die erfindungsgemäßen kosmetischen Mittel gelingt die transparente bis leicht trübe Einarbeitung größerer Mengen von Ölkörpern, die ein hervorragendes Hautgefühl induzieren und trotzdem eine akzeptable Schaummenge zulassen. Sie unterscheiden sich dadurch vorteilhaft von anderen kosmetischen Mittel, die Öle enthalten. So ist der Einsatz des Ölkonzentrates wesentlich für die vorteilhaften Eigenschaften der erfindungsgemäßen kosmetischen Mittel.

### Beispiele

### Beispiel A: Ölkonzentrate

Alle Komponenten (= component) a), b) c1) und ggf. c2) wurden bei Raumtemperatur (23 °C) mit moderater Mechanik homogenisiert. Die erhaltenen Ölkonzentrate wurden visuell bewertet sowie deren Löslichkeit in Wasser geprüft.

Die erfindungsgemäßen Beispiele (= Inv) zeigen alle, dass flüssige anionische Tenside vom Typ a) in Abmischung mit den nichtionischen Tensiden (b) und einem polaren Öl (c1) ggf. in Mischung mit (c2) stets klare, flüssige Ölkonzentrate ergeben, die in Wasser leicht dispergierbar sind unter Bildung einer feinteiligen, stabilen Emulsion.

Im Vergleich dazu (= Comp.) zeigen Ölkonzentrate ohne anionische flüssige Tenside (a) (Vergleichsbeispiel A17) oder ohne nichtionische Tenside (b) (=Vergleichsbeispiel A18) und auch mit anderen flüssigen anionischen Tensiden vom Typ der Lactate (siehe Vergleichsbeispiel A 19) nach mehreren Tagen eine Ausfällung.

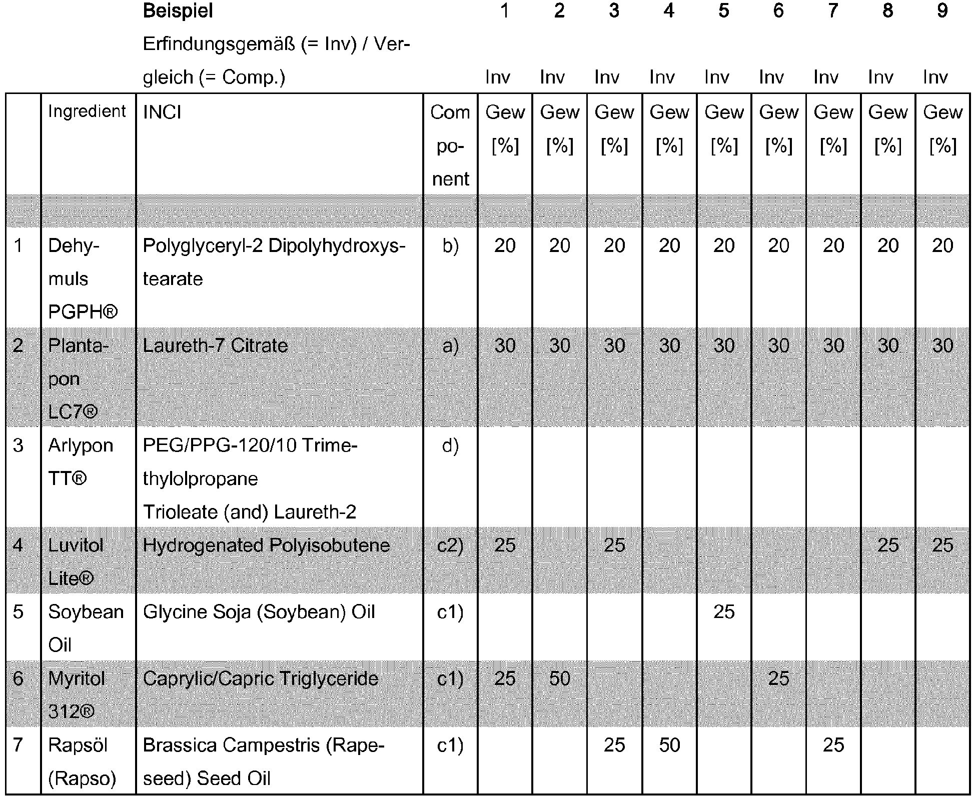

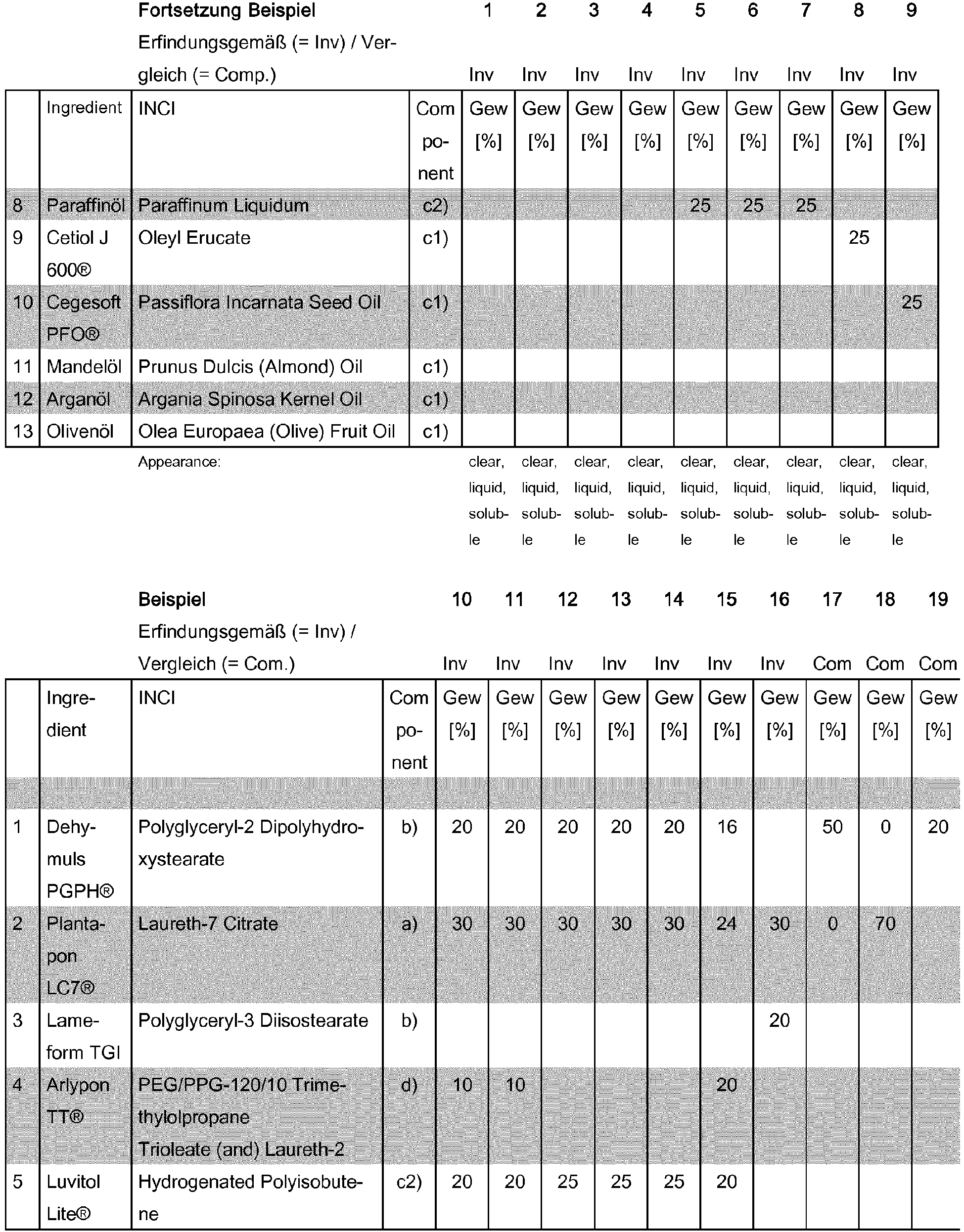

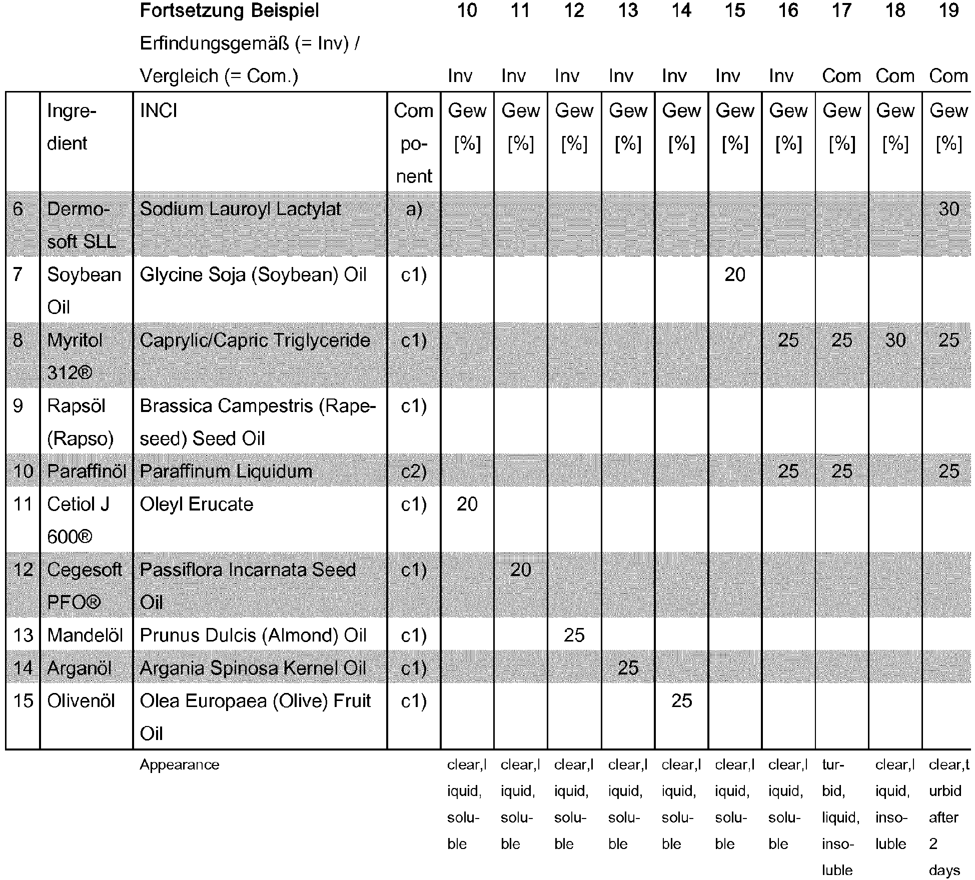

Die Zuordnung "clear" (klar), "turbid" (trübe) und "liquid" (flüssig) erfolgte durch visuelle Betrachtung. Zur Beurteilung von "soluble" (löslich) wurde eine 3 Gew.-%ige wässrige Lösung in Wasser hergestellt, und die Löslichkeit visuell festgestellt.

### Beispiel B: Duschbäder

Wasser 1, Natriumbenzoat sowie Texapon N 70® und Dehyton PK45® werden bei Raumtemperatur homogenisiert, zu der Mischung werden nacheinander bei Raumtemperatur (23°C) das Ölkonzentrat, der Verdicker Arlypon TT® sowie das Wasser 2 eingerührt und anschließend auf das Mittel auf den pH-Wert eingestellt. Die erfindungsgemäßen Duschbäder 20 bis 36 zeigen eine gute Lagerstabilität, d.h. es treten keine Ausfällungen des Öls c1 und ggf. c2 aus den Ölkonzentraten auf. Der Vergleichsversuch 37 zeigt, dass bei direktem Einbringen des Öls ohne Vorformulierung über ein Ölkonzentrat die Lagerstabilität schlecht ist und sich das Öl auch schon bei Raumtemperatur trennt.

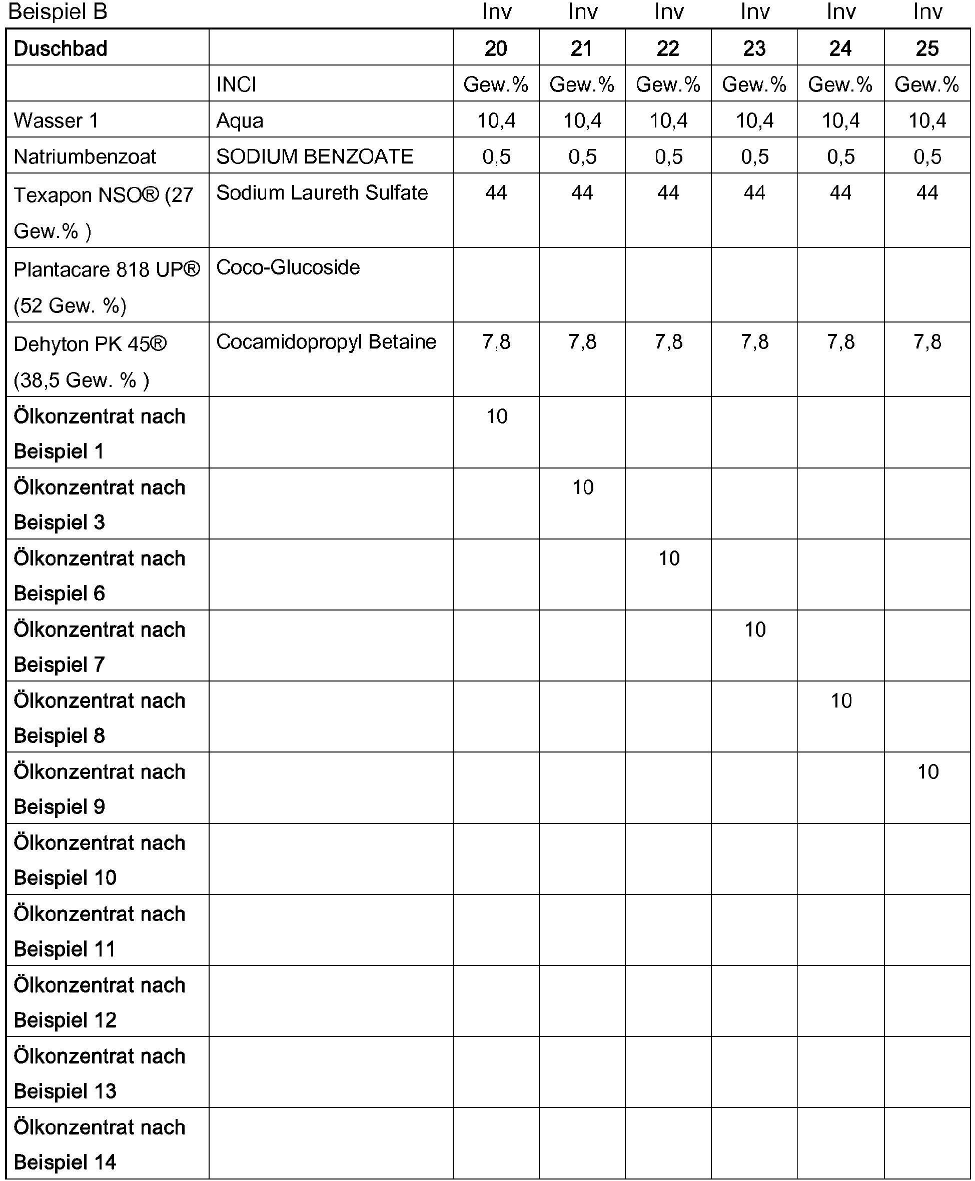

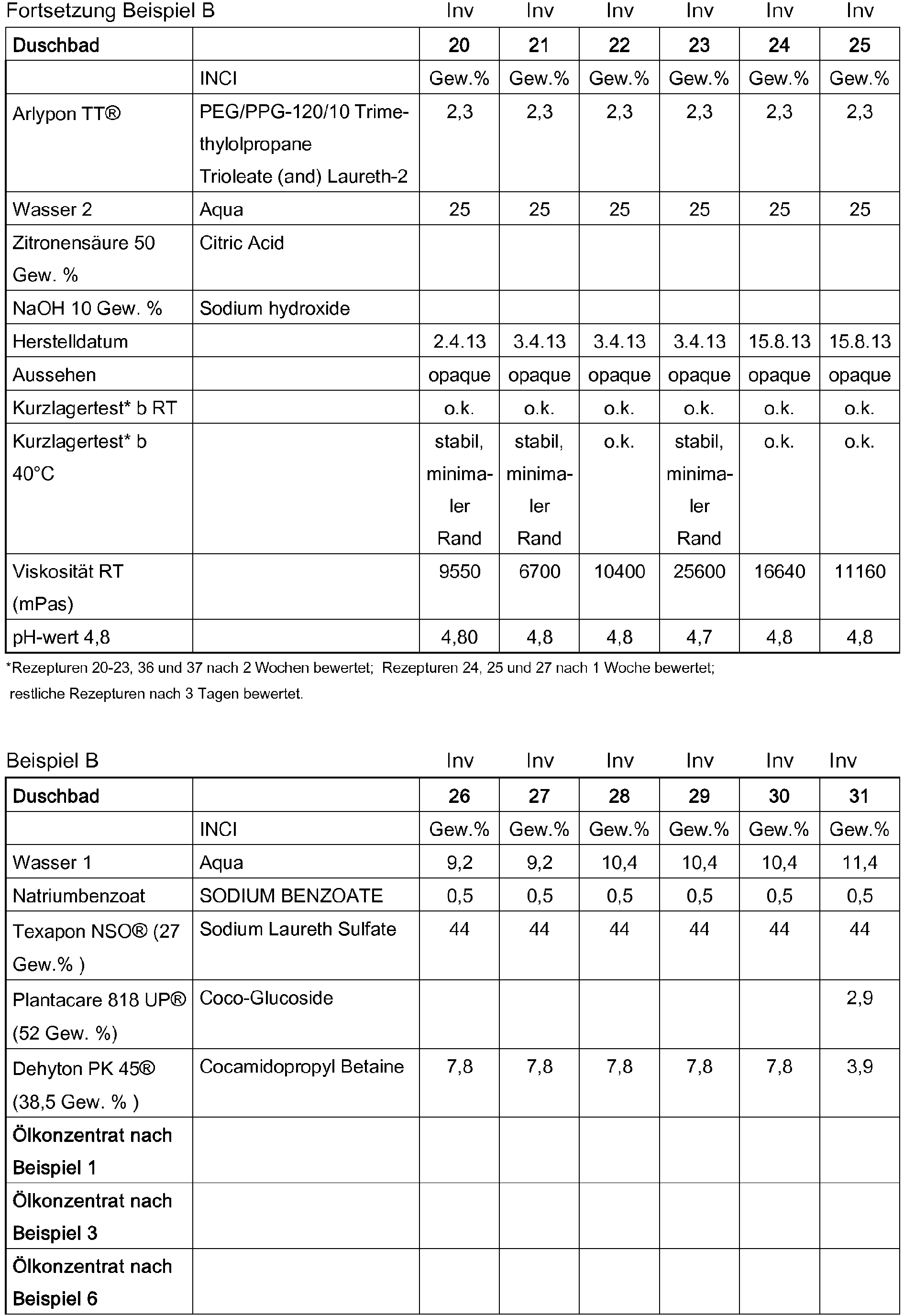

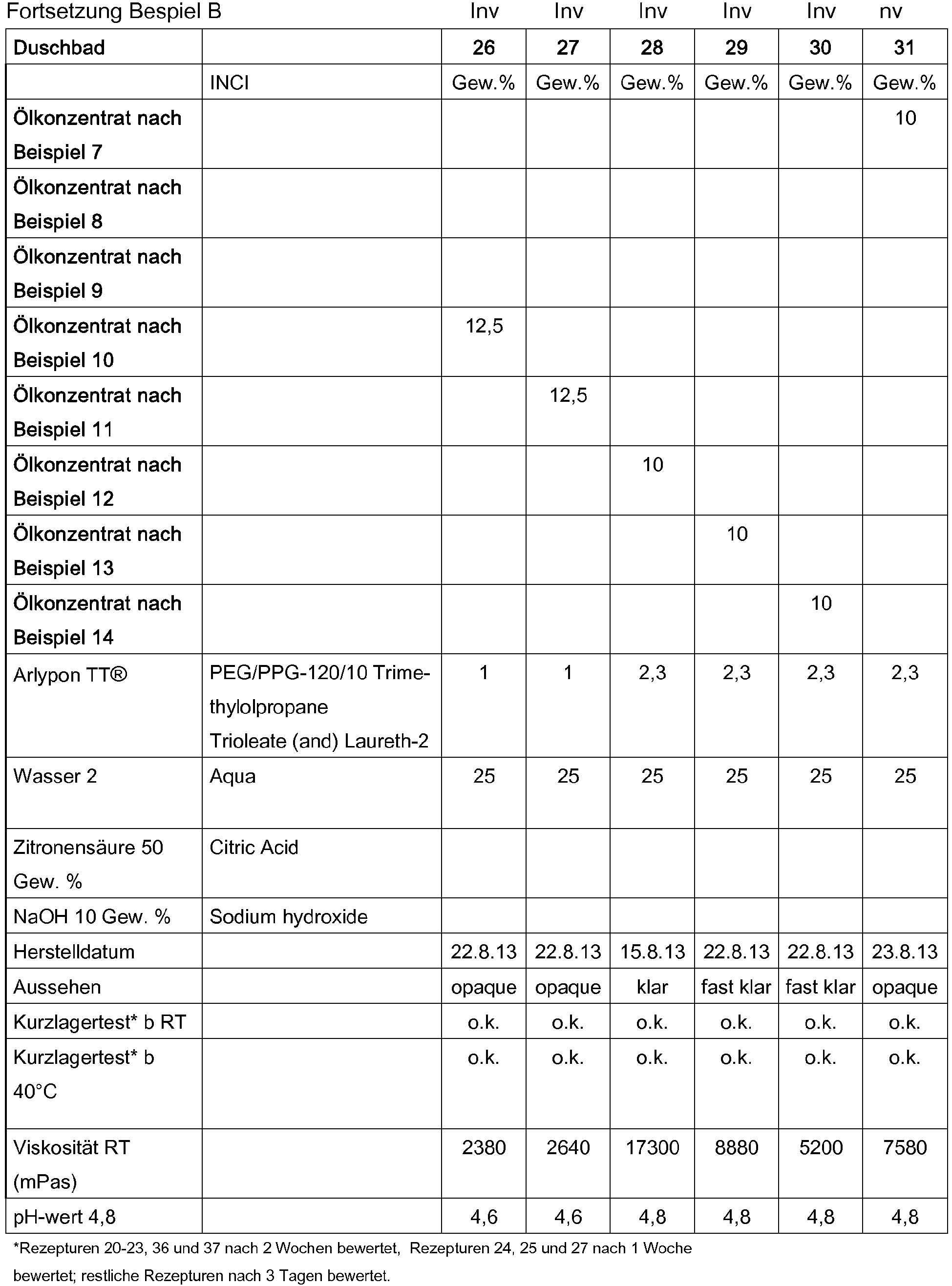

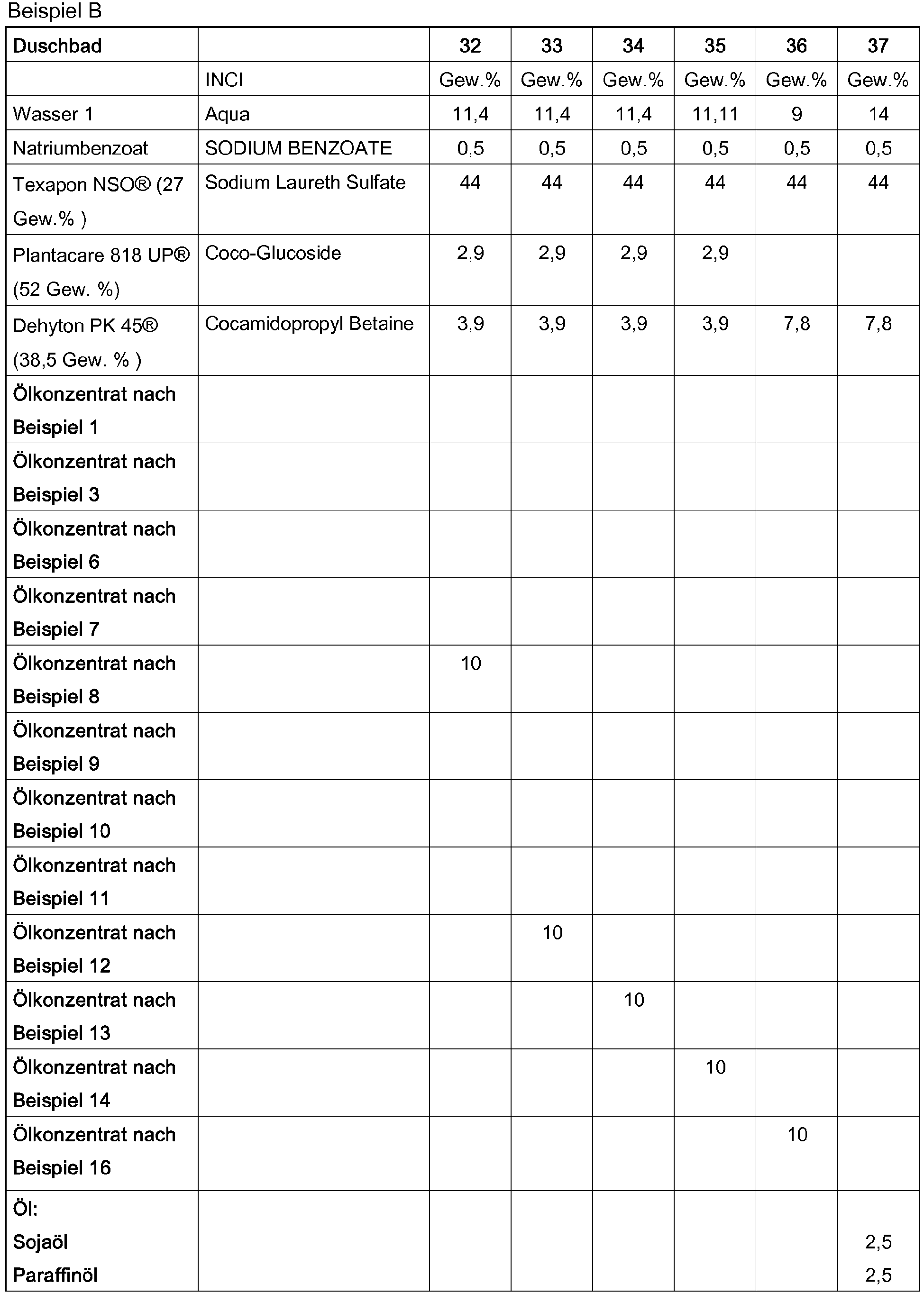

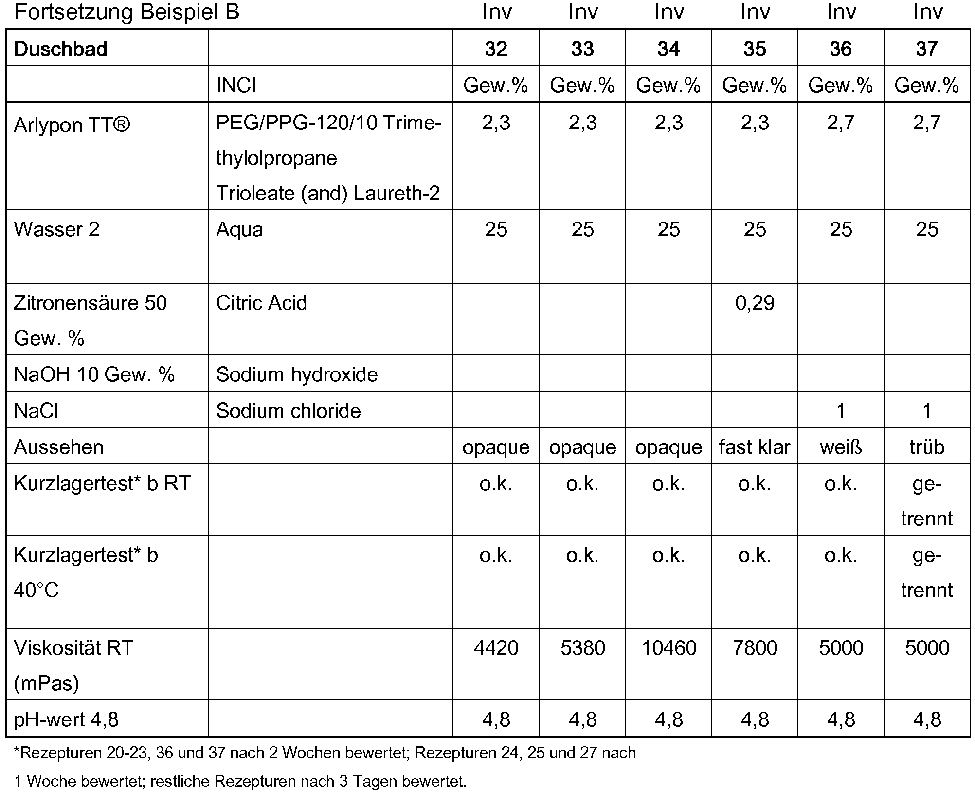

Die Viskosität wurde mit Brookfield bei RT (23 °C) gemessen.

## Patentansprüche

1. Ölkonzentrate für kosmetische Mittel enthaltend
(a) mindestens ein bei Raumtemperatur flüssiges anionisches Tensid ausgewählt aus der Gruppe der Alk(en)ylpolyglykolethercitrate und
(b) mindestens ein nichtionisches Tensid und
(c) eine Ölkomponente mit
(c1) einem polaren Öl mit einer Polarität zwischen 5 und 30 mN/m und ggf.
(c2) einem unpolaren Öl mit einer Polarität über 35 mN/m,
wobei das Mischungsverhältnis (c1): (c1+c2) im Bereich von 0,1 bis 1 liegt und die Ölkomponente (c) in Mengen von 30 bis 60 Gew.-% - bezogen auf das Ölkonzentrat - vorliegt
(d) sowie ggf. Verdickern.

2. Ölkonzentrate nach Anspruch 1, **dadurch gekennzeichnet, dass** sie (a) als anionisches Tensid Alk(en)ylpolyglykolethercitrate der Formel (I) bzw. deren Salze 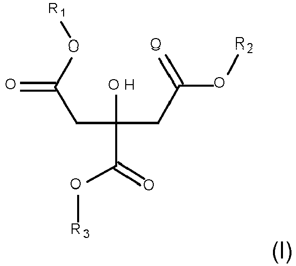 worin
R₁, R₂ und R₃ unabhängig voneinander für Wasserstoff oder den Rest der Formel
(II) R₄(OCH₂CHR₅)ₙ
bedeuten, worin
R₄ für einen linearen oder verzweigten Alkyl- und/oder Alkenylrest mit 6 bis 22 Kohlenstoffatomen,
R₅ für Wasserstoff oder Methylrest und
n für eine Zahl von 1 bis 20 stehen, mit der Bedingung, dass zumindest einer der Reste R₁, R₂, oder R₃ verschieden von Wasserstoff ist, enthalten.

3. Ölkonzentrate nach einem der Ansprüche 1 oder 2, **dadurch gekennzeichnet, dass** sie (a) als anionisches Tensid Alk(en)ylpolyglykolethercitrate der Formel (I), worin R₁, R₂ oder R₃ für den Rest der Formel (II) steht und R₄ einen linearen Alkylrest mit 12 bis 14 Kohlenstoffatome, R₅ Wasserstoff und n eine Zahl von 5 bis 10 bedeutet, enthalten.

4. Ölkonzentrate nach einem der Ansprüche 1 bis 3, **dadurch gekennzeichnet, dass** sie als nichtionisches Tensid (b) Ester von Polyglycerinen, vorzugsweise Ester von Polyglycerinen mit Poly-12-hydroxystearinsäure und/oder Isostearinsäure, enthalten.

5. Ölkonzentrate nach einem der Ansprüche 1 bis 4, **dadurch gekennzeichnet, dass** sie polare Öle (c1) mit einer Polarität zwischen 15 und 28 mN/m enthalten.

6. Ölkonzentrate nach einem der Ansprüche 1 bis 5, **dadurch gekennzeichnet, dass** sie polare Öl (c1) ausgewählt aus der von Olivenöl, Sojaöl, Sonnenblumenöl, Mandelöl, Arganöl und/oder Avocadoöl gebildeten Gruppe enthalten.

7. Ölkonzentrate nach einem der Ansprüche 1 bis 6, **dadurch gekennzeichnet, dass** sie zusätzlich (c2) unpolare Öle mit einer Polarität zwischen 35 und 60 mN/m enthalten.

8. Ölkonzentrate nach einem der Ansprüche 1 bis 7, **dadurch gekennzeichnet, dass** sie Paraffinöle und/oder hydrogenierte Polyisobutene als zusätzliche unpolare Öle (c2) enthalten.

9. Ölkonzentrate nach einem der Ansprüche 1 bis 8, **dadurch gekennzeichnet, dass** das Mischungsverhältnis der polaren Öle : unpolare Öle (c1) : (c1+c2) im Bereich von 0,3 bis 0,7, bevorzugt von 0,4 bis 0,6 und insbesondere von 0,45 bis 0, 55 liegt.

10. Ölkonzentrate nach einem der Ansprüche 1 bis 9, **dadurch gekennzeichnet, dass** sie als Verdicker (d) Mischungen aus ethoxylierten und /oder propoxylierten Polyolestern des Trimethylolpropans und ethoxylierten Fettalkoholen enthalten.

11. Ölkonzentrate nach einem der Ansprüche 1 bis 10, **dadurch gekennzeichnet, dass** sie
(a) Alk(en)ylpolyglykolethercitrate in Mengen von 20 bis 40 Gew.-%, vorzugsweise 25 bis 35 Gew.-% und
(b) mindestens ein nichtionisches Tensid in Mengen von 10 bis 30 Gew.-%, vorzugsweise 15 bis 25 Gew.-% sowie die
(c) Ölkomponente in Mengen von 30 bis 60 Gew.-%, vorzugsweise 40 bis 55 Gew-% sowie ggf.
(d) Verdicker in Mengen von 0 bis 15 Gew.-% enthalten.

12. Kosmetische Mittel, **dadurch gekennzeichnet, dass** sie
A) Ölkonzentrate nach Anspruch 1 und
B) anionische Tenside und
C) weitere von B) verschiedene Co-Tenside und
D) kosmetische Zusatzstoffe und
E) Wasser enthalten.

13. Kosmetische Mittel nach Anspruch 12, **dadurch gekennzeichnet, dass** sie
A) 0,5 bis 20 Gew.-% Ölkonzentrate nach Anspruch 1
B) 5,0 bis 20 Gew.-% anionische Tenside
C) 1 bis 15 Gew.-% weitere von B) verschiedene Co-Tenside
D) 0,1 bis 10 Gew.-% kosmetische Zusatzstoffe und
E) ad 100 Gew.-% Wasser enthalten.

14. Kosmetische Mittel nach einem der Ansprüche 12 oder 13, **dadurch gekennzeichnet, dass** sie
A) 5,0 bis 15 Gew.-% Ölkonzentrate nach Anspruch 1
B) 5,0 bis 20 Gew.-% anionische Tenside ausgewählt aus der von Fettalkoholethersulfat, Monoglycerid(ether)sulfat, Mono- und Dialkylsulfosuccinat, Ethercarbonsäuren bzw. deren Salze, Acylglutamate und Alkyloligoglucosidsulfate gebildeten Gruppe
C) 1 bis 10 Gew.-% amphotere und/oder kationische Tenside
D) 0,5 bis 5,0 Gew.-% kosmetische Zusatzstoffe sowie
E) ad 100 Gew.-% Wasser enthalten.

15. Verfahren zur Herstellung von ölhaltigen kosmetischen Mitteln, **dadurch gekennzeichnet, dass** Ölkonzentrate nach Anspruch 1 mit
B) anionischen Tensiden und
C) nichtionischen Tensiden und
D) kosmetischen Zusatzstoffen und
E) Wasser
in einem Kaltprozess verrührt werden.

## Claims

1. An oil concentrate for cosmetic compositions, comprising
(a) at least one anionic surfactant which is liquid at room temperature and is selected from the group of the alk(en)yl polyglycol ether citrates, and
(b)at least one nonionic surfactant, and
(c)an oil component with
(c1) a polar oil having a polarity of between 5 and 30 mN/m and optionally
(c2) an apolar oil having a polarity of above 35 mN/m, the (c1) : (c1+c2) mixing ratio being in the range from 0.1 to 1 and the oil component (c) being present in amounts of 30 to 60 wt%, based on the oil concentrate,
(d)and also, optionally, thickeners.

2. The oil concentrate according to claim 1, comprising
(a) as anionic surfactant alk(en)yl polyglycol ether citrates of the formula (I) and/or salts thereof 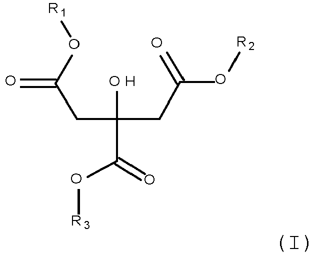 in which
R₁, R₂, and R₃ independently of one another are hydrogen or the radical of the formula
(II) R₄(OCH₂CHR₅)ₙ
in which
R₄ is a linear or branched alkyl and/or alkenyl radical having 6 to 22 carbon atoms,
R₅ is hydrogen or methyl radical, and
n is a number from 1 to 20, subject to the condition that at least one of the radicals, R₁, R₂, or R₃, is other than hydrogen.

3. The oil concentrate according to either of claims 1 and 2, comprising (a) as anionic surfactant alk(en)yl polyglycol ether citrates of the formula (I) in which R₁, R₂, or R₃ is the radical of the formula (II) and R₄ is a linear alkyl radical having 12 to 14 carbon atoms, R₅ is hydrogen, and n is a number from 5 to 10.

4. The oil concentrate according to any of claims 1 to 3, comprising as nonionic surfactant (b) esters of polyglycerols, preferably esters of polyglycerols with poly-12-hydroxystearic acid and/or isostearic acid.

5. The oil concentrate according to any of claims 1 to 4, comprising polar oils (c1) having a polarity of between 15 and 28 mN/m.

6. The oil concentrate according to any of claims 1 to 5, comprising polar oils (c1) selected from the group consisting of olive oil, soybean oil, sunflower oil, almond oil, argan oil and/or avocado oil.

7. The oil concentrate according to any of claims 1 to 6, further comprising (c2) apolar oils having a polarity of between 35 and 60 mN/m.

8. The oil concentrate according to any of claims 1 to 7, comprising liquid paraffins and/or hydrogenated polyisobutenes as additional apolar oils (c2).

9. The oil concentrate according to any of claims 1 to 8, wherein the polar oils : apolar oils (c1) : (c1+c2) mixing ratio is in the range from 0.3 to 0.7, preferably from 0.4 to 0.6, and more particularly from 0.45 to 0.55.

10. The oil concentrate according to any of claims 1 to 9, comprising as thickeners (d) mixtures of ethoxylated and/or propoxylated polyol esters of trimethylolpropane and ethoxylated fatty alcohols.

11. The oil concentrate according to any of claims 1 to 10, comprising
(a)alk(en)yl polyglycol ether citrates in amounts of 20 to 40 wt%, preferably 25 to 35 wt%, and
(b)at least one nonionic surfactant in amounts of 10 to 30 wt%, preferably 15 to 25 wt%, and also the
(c) oil component in amounts of 30 to 60 wt%, preferably 40 to 55 wt%, and also, optionally,
(d) thickeners in amounts of 0 to 15 wt%.

12. A cosmetic composition comprising
A) oil concentrate according to claim 1 and
B) anionic surfactants and
C) further, co-surfactants different from (B), and
D) cosmetic additives, and
E) water.

13. The cosmetic composition according to claim 12, comprising
A) 0.5 to 20 wt% of oil concentrate according to claim 1
B) 5.0 to 20 wt% of anionic surfactants
C) 1 to 15 wt% of further, co-surfactants different from
D) 0.1 to 10 wt% of cosmetic additives, and
E) water ad 100 wt%.

14. The cosmetic composition according to either of claims 12 and 13, comprising
A) 5.0 to 15 wt% of oil concentrate according to claim 1
B) 5.0 to 20 wt% of anionic surfactants selected from the group consisting of fatty alcohol ether sulfate, monoglyceride ether sulfate, mono- and dialkylsulfosuccinate, ethercarboxylic acids and/or salts thereof, acylglutamates, and alkyloligoglucoside sulfates
C) 1 to 10 wt% of amphoteric and/or cationic surfactants
D) 0.5 to 5.0 wt% of cosmetic additives, and
E) water ad 100 wt%.

15. A method for producing oil-containing cosmetic compositions, which comprises stirring together oil concentrate according to claim 1 with
B) anionic surfactants and
C) nonionic surfactants and
D) cosmetic additives, and
E) water
in a cold operation.

## Revendications

1. Concentrés huileux pour agents cosmétiques, contenant :
(a) au moins un tensioactif anionique liquide à température ambiante choisi dans le groupe des citrates d'éther d'alkyl- ou alcénylpolyglycol, et
(b) au moins un tensioactif non ionique et
(c) un composant huileux contenant
(c1) une huile polaire ayant une polarité comprise entre 5 et 30 mN/m et éventuellement
(c2) une huile apolaire ayant une polarité supérieure à 35 mN/m,
le rapport de mélange (c1):(c1+c2) se situant dans la plage allant de 0,1 à 1, et le composant huileux (c) étant présent en quantités de 30 à 60 % en poids par rapport au concentré huileux, et
(d) éventuellement des épaississants.

2. Concentrés huileux selon la revendication 1, **caractérisés en ce qu'**ils contiennent (a) en tant que tensioactif anionique des citrates d'éther d'alkyl- ou alcénylpolyglycol de formule (I) ou leurs sels 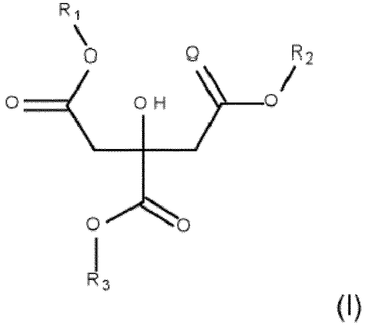 dans laquelle
R₁, R₂ et R₃ signifient indépendamment les uns des autres l'hydrogène ou le radical de formule
(II) R₄(OCH₂CHR₅)ₙ
dans laquelle
R₄ représente un radical alkyle et/ou alcényle linéaire ou ramifié de 6 à 22 atomes de carbone,
R₅ représente l'hydrogène ou un radical méthyle, et
n représente un nombre de 1 à 20, à condition qu'au moins un des radicaux R₁, R₂ ou R₃ soit différent de l'hydrogène.

3. Concentrés huileux selon l'une quelconque des revendications 1 ou 2, **caractérisés en ce qu'**ils contiennent (a) en tant que tensioactif anionique des citrates d'éther d'alkyl- ou alcénylpolyglycol de formule (I), dans laquelle R₁, R₂ ou R₃ représente le radical de formule (II), et R₄ signifie un radical alkyle linéaire de 12 à 14 atomes de carbone, R₅ signifie l'hydrogène et n signifie un nombre de 5 à 10.

4. Concentrés huileux selon l'une quelconque des revendications 1 à 3, **caractérisés en ce qu'**ils contiennent en tant que tensioactif non ionique (b) des esters de polyglycérine, de préférence des esters de polyglycérine avec de l'acide poly-12-hydroxystéarique et/ou de l'acide isostéarique.

5. Concentrés huileux selon l'une quelconque des revendications 1 à 4, **caractérisés en ce qu'**ils contiennent des huiles polaires (c1) ayant une polarité comprise entre 15 et 28 mN/m.

6. Concentrés huileux selon l'une quelconque des revendications 1 à 5, **caractérisés en ce qu'**ils contiennent une huile polaire (c1) choisie dans le groupe formé par l'huile d'olive, l'huile de soja, l'huile de tournesol, l'huile d'amande, l'huile d'argan et/ou l'huile d'avocat.

7. Concentrés huileux selon l'une quelconque des revendications 1 à 6, **caractérisés en ce qu'**ils contiennent en outre (c2) des huiles apolaires ayant une polarité comprise entre 35 et 60 mN/m.

8. Concentrés huileux selon l'une quelconque des revendications 1 à 7, **caractérisés en ce qu'**ils contiennent des huiles de paraffine et/ou des polyisobutènes hydrogénés en tant qu'huiles apolaires supplémentaires (c2).

9. Concentrés huileux selon l'une quelconque des revendications 1 à 8, **caractérisés en ce que** le rapport de mélange huiles polaires:huiles apolaires (c1):(c1+c2) se situe dans la plage allant de 0,3 à 0,7, de préférence de 0,4 à 0,6 et notamment de 0,45 à 0,55.

10. Concentrés huileux selon l'une quelconque des revendications 1 à 9, **caractérisés en ce qu'**ils contiennent en tant qu'épaississant (d) des mélanges d'esters de polyol éthoxylés et/ou propoxylés de triméthylolpropane et d'alcools gras éthoxylés.

11. Concentrés huileux selon l'une quelconque des revendications 1 à 10, **caractérisés en ce qu'**ils contiennent
(a) des citrates d'éther d'alkyl- ou alcénylpolyglycol en quantités de 20 à 40 % en poids, de préférence de 25 à 35 % en poids, et
(b) au moins un tensioactif non ionique en quantités de 10 à 30 % en poids, de préférence de 15 à 25 % en poids, et
(c) le composant huileux en quantités de 30 à 60 % en poids, de préférence de 40 à 55 % en poids, et éventuellement
(d) des épaississants en quantités de 0 à 15 % en poids.

12. Agents cosmétiques, **caractérisés en ce qu'**ils contiennent
A) des concentrés huileux selon la revendication 1 et
B) des tensioactifs anioniques, et
C) d'autres co-tensioactifs différents de B), et
D) des additifs cosmétiques et
E) de l'eau.

13. Agents cosmétiques selon la revendication 12, **caractérisés en ce qu'**ils contiennent
A) 0,5 à 20 % en poids de concentrés huileux selon la revendication 1,
B) 5,0 à 20 % en poids de tensioactifs anioniques,
C) 1 à 15 % en poids d'autres co-tensioactifs différents de B),
D) 0,1 à 10 % en poids d'additifs cosmétiques et
E) jusqu'à 100 % en poids d'eau.

14. Agents cosmétiques selon l'une quelconque des revendications 12 ou 13, **caractérisés en ce qu'**ils contiennent
A) 5,0 à 15 % en poids de concentrés huileux selon la revendication 1,
B) 5,0 à 20 % en poids de tensioactifs anioniques choisis dans le groupe formé par les éther-sulfates d'alcools gras, les (éther)sulfates de monoglycérides, les sulfosuccinates de mono- et dialkyle, les acides éthercarboxyliques et leurs sels, les glutamates d'acyle et les sulfates d'alkyloligoglucoside,
C) 1 à 10 % en poids de tensioactifs amphotères et/ou cationiques,
D) 0,5 à 5,0 % en poids d'additifs cosmétiques et
E) jusqu'à 100 % en poids d'eau.

15. Procédé de fabrication d'agents cosmétiques contenant de l'huile, **caractérisé en ce que** des concentrés huileux selon la revendication 1 sont agités dans un procédé à froid avec
B) des tensioactifs anioniques et
C) des tensioactifs non ioniques et
D) des additifs cosmétiques et
E) de l'eau.
